# EUROPÄISCHE PATENTSCHRIFT

(11) **EP 1 211 994 B1**
(45) Veröffentlichungstag und Bekanntmachung des Hinweises auf die Patenterteilung: **27.04.2005**
(21) Anmeldenummer: 00964173.9
(22) Anmeldetag: 14.09.2000
(51) Int. Cl.: A61B 17/80

(54) **FIXATIONSSYSTEM FÜR KNOCHEN**
FIXING SYSTEM FOR BONES
SYSTEME DE FIXATION DES OS

(30) Priorität: 14.09.1999 DE 19943924; 23.12.1999 DE 19962317
(43) Veröffentlichungstag der Anmeldung: 12.06.2002
(73) Patentinhaber: Wolter, Dietmar, Prof. Dr., D-21033 Hamburg (DE)
(72) Erfinder: WOLTER, Dietmar Berufsgenossenschaftliches, 21033 Hamburg (DE); SCHÜMANN, Uwe Berufsgenossenschaftliches, 21033 Hamburg (DE); SEIDE, Klaus, 21465 Reinbek (DE)
(74) Vertreter: Siemons, Norbert, Dr.-Ing.
(86) Internationale Anmeldenummer: PCT/EP2000/008999
(87) Internationale Veröffentlichungsnummer: WO 2001/019264

(56) Entgegenhaltungen:
- WO-A-98/09578
- DE-A- 4 343 117
- DE-A- 19 629 011
- FR-A- 742 618
- US-A- 3 463 148
- US-A- 4 683 878

## Beschreibung

Die Erfindung bezieht sich auf ein Fixationssystem für Knochen mit einem Kraftträger mit Löchern und in die Löcher einsetzbaren und fixierbaren Knochenschrauben.

Müssen Knochenbruchstücke miteinander verbunden werden, so stehen heute Platten-, Nagel- und Fixateursysteme zur Verfügung. Bisher wurde bei Platten- und Nagelsystemen die Stabilität dadurch erreicht, daß bei den Osteosyntheseplatten Knochenschrauben diese Platten fest an den Knochen heranzogen, um so durch den Anpreßdruck der Platte an den Knochen eine Stabilisierung der Knochenbruchstücke zu erreichen. Bei dem Marknagel, der im Markraum liegt, wird die Stabilität ebenfalls dadurch erhöht, daß Schrauben quer durch Knochen und Marknagel eingebracht werden. Diese Schrauben liegen zwar im Knochen mit einem Gewinde fest, die Durchquerung der Schraube durch den Nagel läßt jedoch kleinere Bewegungen zu.

Aus der US 4 683 878 ist eine Fixationsplatte für die Osteosynthese der vorerwähnten Art bekannt. Diese hat einen Zentralabschnitt, dessen Breite deutlich geringer ist als die Breite der Endabschnitte. Die Endabschnitte der Befestigungsplatte haben eine Mehrzahl von länglichen Löchern. Die Dicke jedes Endabschnittes nimmt ausgehend vom Zentralabschnitt zu den äußeren Enden der Befestigungsplatte hin ab. Die Mehrzahl der länglichen Löcher hat eine vertikale distale Begrenzungswand und eine geneigte proximale Begrenzungswand, die sich zur vertikalen Begrenzungswand hin neigt. Jeder Endabschnitt hat eine gleichmäßige Breite und die gesamte Unterseite jedes Endabschnittes kann konkav geformt sein, um eine komplementäre Gegenfläche zu dem gebrochenen Knochen zur Verfügung zu stellen. Durch Einsatz der Knochenschrauben mit einem Kopf, dessen Unterseite gerundet ist, zieht die Befestigungsplatte die Knochenstücke zusammen und hält die Knochenstücke unter Druck an der Bruchstelle, wenn die Platte und die Schrauben vollständig an dem Knochen befestigt sind.

Durch unterschiedliche technische Lösungen ist es gelungen, den Schraubenkopf mit der Platte fest zu verbinden bzw. eine feste Verbindung zwischen der Schraube und dem Marknagel selbst herzustellen. Hierzu wird auf die EP 0 201 024 B1, DE 43 43 117 A1, DE 196 29 011 A1 und die deutsche Patentanmeldung P 198 58 889.5 Bezug genommen.

Bei dieser neuen Generation von Implantaten kann man daher von inneren Fixateursystemen sprechen, da das Hauptmerkmal der äußeren Fixateure die Winkelstabilität zwischen Schraube und dem queren Kraftträger ist.

In der klinischen Anwendung zeigt sich bisher deutlich eine Überlegenheit dieser Fixateur intem-Systeme gegenüber herkömmlichen Platten- und Nagelsystemen.

Belastet der Patient jedoch entgegen ärztlichem Rat zu früh mit zu viel Körpergewicht, so kann es zur Verbiegung des Implantates bzw. zum Implantatbruch kommen.

Außerdem ist beobachtet worden, daß es bei weichen Knochen und einer hohen Biegebelastung zum Herausreißen der Schrauben aus dem Knochen kommen kann.

Davon ausgehend liegt der Erfindung die Aufgabe zugrunde, das Fixationssystem für Knochen gemäß erstgenannter Druckschrift hinsichtlich der Belastungseffekte zu verbessern.

Die Aufgabe wird durch ein Fixationssystem für Knochen mit den Merkmalen des Anspruches 1 gelöst. Vorteilhafte Ausgestaltungen der Fixationssystems sind in den Ansprüchen 2 bis 11 angegeben.

Der Erfindung liegt die überraschende Erkenntnis zugrunde, daß das Schraubenloch, welches am nächsten zur Bruch- oder Instabilitätszone eines Knochens liegt, der höchsten Belastung unterworfen ist und daß die Versagensursache in diesem Bereich liegt.

Vergleicht man die Mechanik herkömmlicher, nicht winkelstabiler Plattensysteme mit winkelstabilen Fixateur inteme-Systemen, so läßt sich bei Belastung überraschenderweise ein grundsätzlicher Unterschied erkennen. Auch bei nicht winkelstabilen Plattensystemen finden wir Plattenbrüche, die in der Regel durch ein Plattenloch gehen bzw. zu Lockerungserscheinungen mit Heraustreten der Schrauben aus dem Knochen führen. Bei winkelstabilen Systemen kommt es aufgrund der festen Verankerung des Schraubenkopfes in der Platte bzw. der Schraube im Marknagel nicht zu einer Veränderung der Schraubenposition zur Platte selbst oder zum Marknagel. Dies bedeutet, daß einwirkende Kräfte sich nicht gleichmäßig auf eine Deformation der Platte oder des Nagels auswirken, wie sie mehr oder weniger bei nicht winkelstabilen Implantaten zu erkennen ist, sondern daß diese Kräfte zu einer verstärkten Biegebelastung in dem Bereich des Schraubenloches führen, das am benachbartesten zur Bruch- oder Instabilitätszone zu liegen kommt. Auch wenn bei sachgerechtem und korrektem Verhalten des Patienten ein regelrechter Heilungsverlauf bei herkömmlich dimensionierten winkelstabilen Implantaten erfolgt, so ist der Bruch an dieser Stelle bei fehlerhafter Überbeanspruchung ein Hinweis darauf, daß hier die einwirkenden Kräfte zu einer Schädigung führen. Dabei kann es dann zu einem Implantatversagen kommen.

Deshalb sieht die Erfindung eine Verstärkung des Kraftträgers an dem Loch vor, das in der Nähe der Bruch- oder Instabilitätszone eines Knochens anzuordnen ist. An weiter entfernt anzuordnenden Löchern braucht der Kraftträger keine Verstärkung aufzuweisen. Da man jedoch davon ausgehen kann, daß der Kraftträger nicht nur am ersten Loch stärker beansprucht wird, sondern auch das folgende Loch eine höhere Belastung aufnehmen muß, wird bevorzugt auch das nächstfolgende Loch mit einer Verstärkung versehen, die jedoch deutlich geringer dimensioniert sein kann. So kann das angrenzende Loch eine etwa um die Hälfte reduzierte Verstärkung haben. Die weiter entfemten Löcher können jedoch in der Regel als unkritisch angesehen werden und bedürfen daher zumeist keiner Verstärkung.

Die Verstärkung des Kraftträgers kann unterschiedlich erfolgen:
1. durch Querschnittsvergrößerung des Kraftträgers, insbesondere
   1.1 durch Verdickung des Kraftträgers (in Lochrichtung) und/oder
   1.2 durch Verbreiterung des Kraftträgers (quer zur Lochrichtung), die zum Beispiel bogenförmig nach außen verlaufen kann, und/oder
   1.3 durch Verringerung der Querausdehnung (insbesondere eines Durchmessers des Loches und/oder
2. durch einen festeren Werkstoff des Kraftträgers im Lochbereich.

Bei bevorzugten Ausgestaltungen des Fixationssystems sind die Knochenschrauben unter verschiedenen Winkeln in die Löcher des Kraftträgers einsetzbar und in den Löchern fixierbar. Hierfür können Kraftträger bzw. Knochenschrauben gemäß den eingangs genannten Patentanmeldungen ausgestaltet sein, insbesondere gemäß DE 43 43 117 A1, DE 196 29 011 A1 und P 198 58 889.5.

Der Ausgestaltung gemäß Anspruch 5 liegt die überraschende Erkenntnis zugrunde, daß Knochenschrauben insbesondere dann ausreißgefährdet sind, wenn sie parallel zueinander in den Knochen eingebracht werden. Um dieses Heraustreten aus dem Knochen zu vermeiden,
werden mindestens zwei Löcher nicht parallel zueinander in den Kraftträger eingebracht, sondern schräg zueinander geneigt. Dafür kann gegenüber herkömmlichen Kraftträgern, bei denen die Löcher in einem Winkel von 90° zum Kraftträger (bzw. zu einer Zentralebene bzw. einer Auflageebene desselben am Knochen) eingebracht sind, mindestens ein Loch schräg zum Kraftträger geneigt sein. Vorzugsweise können zwei oder mehrere Löcher entsprechend zueinander geneigt im Kraftträger angeordnet sein. Bevorzugt wird dabei, daß Löcher, die auf verschiedenen Seiten einer Bruch- oder Instabilitätszone eines Knochens anzuordnen sind, in verschiedenen Richtungen zueinander geneigt im Kraftträger angeordnet sind.

Da der Knochen in der Regel gebogene Oberflächen aufweist und dieses insbesondere im gelenknahen Bereich der Fall ist, besteht die Notwendigkeit, daß insbesondere Plattensysteme dieser Knochenbiegung angepaßt werden. Dieser Vorgang erfolgt in der Regel durch entsprechende Biegewerkzeuge während der Operation. Dabei kann auch die Ausrichtung von Plattenlöchern entsprechend der Anmodellierung verändert werden. Findet sich eine deutliche Knochenoberflächenbiegung, wie im gelenknahen Bereich, so kann die schräge Anlegung des Schraubenloches das Erreichen einer optimalen Schraubenlage im Knochen weiter erleichtern. Dies kann bei der Ausrichtung von Löchern in der Platte von vornherein berücksichtigt werden, so daß nach dem Anmodellieren eine gewünschte schräge Ausrichtung mindestens zweier Löcher in der Platte erreicht wird.

Vorzugsweise können die Knochenschrauben unter verschiedenen Winkeln in die Löcher des Kraftträgers einsetzbar und in diesem fixierbar sein. Dabei können Kraftträger bzw. Schrauben gemäß den eingangs erwähnten Patentanmeldungen ausgestaltet sein, insbesondere gemäß DE 43 43 117 A1, DE 196 29 011 A1 oder P 198 58 889.5. Dadurch, daß mindestens zwei Löcher im Kraftträger schräg zueinander geneigt sind, ist es möglich, mindestens zwei Knochenschrauben von vornherein zueinander geneigt in den Kraftträger einzubringen, ohne den durch die Fixierbarkeit unter verschiedenen Winkeln gegebenen Spielraum zu verbrauchen. Hierdurch werden die Möglichkeiten, durch Schräglage eine Verspreizung der Schrauben im Knochen zu erreichen, erheblich verbessert.

Das Fixationssystem kann insbesondere eine Knochenplatte, ein Knochennagel oder ein Fixateur sein.

Durch die Optimierung der Lochgestaltung im Hinblick auf eine dem Kraftfluß adaptierte Querschnittsvergrößerung bzw. Werkstoffauswahl bzw. durch die schräg ausgerichteten Löcher läßt sich ein möglicher Bruch des Kraftträgers bei unsachgemäßem Patientenverhalten bzw. ein Ausreißen des Implantates ebenfalls bei Überbeanspruchung vermeiden.

Die Erfindung wird nachfolgend anhand der anliegenden Zeichnungen von Ausführungsbeispielen näher erläutert. In den Zeichnungen zeigen:
Fig. 1 eine Knochenplatte mit Verbreiterungen in teilweiser Draufsicht;
Fig. 2 eine Knochenplatte mit Verdickungen in einem teilweisen Längsschnitt;
Fig. 3 eine gebogene Knochenplatte an einem Tibia-Knochen in Gelenksnähe im teilweisen Längsschnitt;
Fig. 4 eine Knochenplatte mit geneigten Löchern am Mittelbereich eines Röhrenknochens im Längsschnitt;
Fig. 5 eine Knochenplatte mit Löchern unterschiedlichen Durchmessers am Mittelbereich eines Röhrenknochens im Längsschnitt.

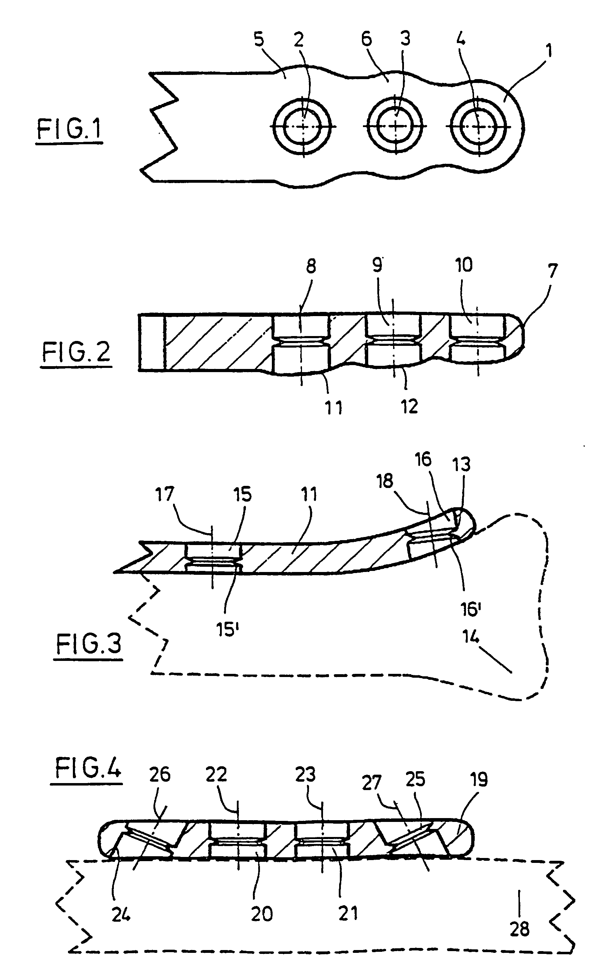

Gemäß Fig. 1 hat eine Knochenplatte 1 in einem Abschnitt drei Plattenlöcher 2, 3, 4. Davon ist das Plattenloch 2 am nächsten an einer Bruch- oder Instabilitätszone eines Knochens anzuordnen, das Loch 3 weiter entfernt anzuordnen und das Loch 4 am weitesten davon entfernt anzuordnen. Um das Loch 2 hat die Knochenplatte 1 eine Verstärkung in Form einer großen Verbreiterung 5. Am Loch 3 ist ebenfalls eine Verstärkung in Form einer Verbreiterung 6 vorhanden, die jedoch nur halb so groß ist, wie die Verbreiterung 5. Am Loch 4 ist keine Verbreiterung mehr vorhanden, sondern hat die Knochenplatte eine im wesentlichen konstante Breite. Die Verbreiterungen 5, 6 bestehen jeweils aus Ausbiegungen auf beiden Seiten der Knochenplatte 1.

In dem abgeschnittenen Bereich (in der Zeichnung links), der auf der anderen Seite der Bruch- oder Instabilitätszone eines Knochens anzuordnen ist, kann eine entsprechende Lochfolge mit entsprechender Verstärkung vorhanden sein.

Fig. 2 zeigt eine Knochenplatte 7, deren Löcher 8, 9, 10 entsprechend zu einer Bruch- oder Instabilitätszone eines Knochens anzuordnen sind. Deshalb ist sie im Bereich des Loches 8 in einer maximalen Verdickung 11 ausgeführt, im Bereich des Loches 9 mit einer etwa nur halb so großen Verdickung 12 und im Bereich des Loches 10 weist sie keine Verdickung auf.

Eine Knochenplatte (oder ein anderer Kraftträger) kann auch in Kombination Verbreiterungen 5, 6 entsprechend Fig. 1 und Verdickungen 11, 12 gemäß Fig. 2 ausweisen.

Gemäß Fig. 3 ist eine Knochenplatte 13 durch Verbiegen so verformt, daß sie gut an den Gelenksbereich eines Tibia-Knochens 14 paßt. Sie weist Löcher 15, 16 auf. Auf dem abgeschnittenen Teil der Knochenplatte 13 können weitere Löcher vorhanden sein. Die Achse des Loches 15 ist senkrecht zur Knochenplatte 13 ausgerichtet. Die Achse des Loches 16 ist von vornherein zur Knochenplatte bzw. deren Auflagefläche auf dem Knochen geneigt. Dabei ist die Neigung der Achse des Loches 16 so geplant, daß nach deren Anmodellieren an den Knochen 14 eine Schrägausrichtung der Achsen der Löcher 15, 16 zueinander vorhanden ist. Dies führt zu einer Verspreizung eingedrehter Schrauben im Knochen, die einem Ausreißen des Implantates aus dem Knochen entgegenwirkt.

Gemäß Fig. 3 (und auch bei allen weiteren Ausführungsbeispielen) sind die Löcher 15, 16 der Knochenplatte 11 an ihrem Innenumfang mit einem umlaufenden Grat 15', 16' versehen. In diesen Grat 15', 16' kann eine Knochenschraube mit einem Gewinde an der Unterseite ihres Kopfes in verschiedenen Winkelstellungen eingedreht werden, wobei eine Umformung des Grates 15', 16' eintritt, je nachdem, in welchem Winkel zur Achse des Loches 15, 16 die Knochenschraube eingedreht wird. Zudem wird bei der Umformung des Grates bzw. des Gewindes der Schraube eine Sicherung der Schraube in der Eindrehstellung des Knochens bewirkt. Durch die vorgeplante Schräglage der Achsen der Löcher 15, 16 zueinander ist eine geneigte Ausrichtung der Knochenschrauben zueinander möglich, ohne den für die Fixierbarkeit unter verschiedenem Winkel in der Knochenplatte 11 gegebenen Spielraum zu verbrauchen. Somit wird zugleich eine Verspreizung und eine optimale Ausrichtbarkeit der Knochenschrauben in einer individuell optimierbaren Winkellage in ihren Löchern 15, 16 erreicht.

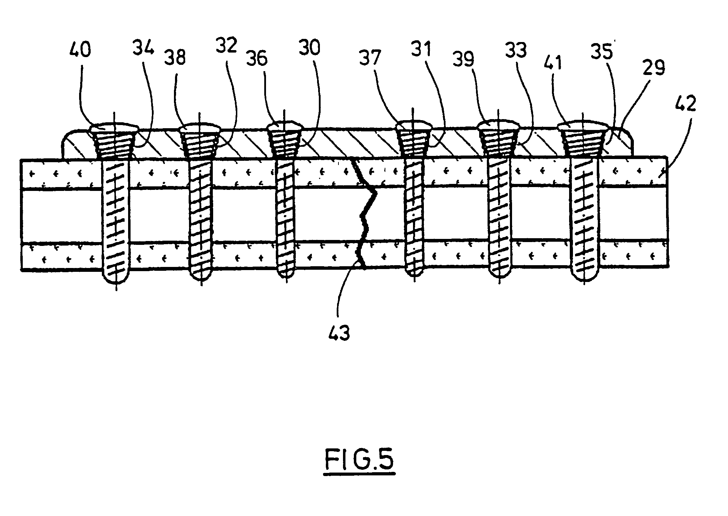

Fig. 4 zeigt eine Knochenplatte 19, bei der die beiden mittleren Löcher 20, 21 mit ihren Achsen 22, 23 konventionell senkrecht zur Knochenplatte ausgerichtet sind. Die beiden äußeren Löcher 24, 25 sind jedoch mit ihren Achsen 26, 27 spitzwinklig zur Knochenplatte 18 ausgerichtet. Infolgedessen wird eine Verspreizung der in die beiden äußeren Löcher 24, 25 einzudrehenden Knochenschrauben in einem angrenzenden Knochen 28 erreicht und damit eine sicherere Befestigung. Auch bei diesem Anwendungsbeispiel ist eine Fixierbarkeit der Knochenschrauben unter verschiedenen Winkeln in den Löchern 20, 21, 24, 25 gegeben.

Fig. 5 zeigt eine Knochenplatte 29, die konische Löcher 30 bis 35 aufweist. Diese verjüngen sich von der Oberseite zur Unterseite der Knochenplatte 29 hin.

Die beiden Löcher 30, 31, die dem Schwerpunkt der Knochenplatte 29 am nächsten liegen, haben kleinere Abmessungen als die beiden weiter außen liegenden Löcher 32, 33. Letztere haben wiederum kleinere Abmessungen als die ganz außen liegenden Löcher 34, 35. Dabei haben im gezeigten Beispiel die mittleren Löcher 30, 31 identische Abmessungen. Ferner haben die weiter außen liegenden Löcher 32, 33 übereinstimmende Abmessungen. Schließlich hat auch das Paar Löcher 34, 35 übereinstimmende Abmessungen.

Infolge der geschilderten Größenverteilung der Löcher 30, 35 hat die Knochenplatte 29 im Bereich der Löcher 30, 31 den größten Querschnitt, im Bereich der Löcher 32, 33 einen etwas kleineren Querschnitt und im Bereich der Löcher 34, 35 den kleinsten Querschnitt. Dabei kann die Knochenplatte 29 überall mit konstanter Dicke und Breite ausgeführt sein. Grundsätzlich kann aber auch eine Verbreiterung und/oder eine Verdickung gemäß den Ausführungen von Fig. 1 und 2 hinzukommen.

Die Knochenplatte 29 ist mittels eingedrehter Knochenschrauben 36 bis 41 so an einem Röhrenknochen 42 fixiert, daß eine Bruchzone genau zwischen den beiden zentralen Löchern 30, 31 angeordnet ist. Die über die Knochenschrauben 36 bis 41 in die Knochenplatte 29 eingeleitete Kraft ist um so größer, je näher die jeweilige Knochenschraube an der Bruchzone 43 liegt. Dem entspricht die Knochenplatte 29 festigkeitsmäßig dadurch, daß sie einen um so größeren Querschnitt aufweist, je näher die Löcher 30 bis 35 an der Bruchzone 43 liegen.

Die Knochenschrauben 36 bis 41 haben übrigens oberhalb ihres Gewindeschaftes an der Unterseite ihres Kopfes jeweils ein konisches Gewinde, das in das jeweilige Loch 30 bis 35 eingedreht wird. Infolge der Konizität ist ein Eindrehen unter unterschiedlichen Winkelausrichtungen möglich. Dabei gräbt sich das Gewinde an der Unterseite des Kopfes in die Innenfläche des jeweiligen Loches 30 bis 35 ein, wodurch eine Fixierung der Eindreh-Winkellage erzielt wird. In der Fig. 5 ist allerdings eine Ausrichtung sämtlicher Knochenschrauben 36 bis 41 in einem Winkel von 90° zur Ebene der Knochenplatte 29 gezeigt.

## Patentansprüche

1. Fixationssystem für Knochen mit einem Kraftträger (1) mit Löchern (2, 3, 4) und in die Löcher (2, 3, 4) einsetzbaren Knochenschrauben (36 bis 41), bei dem der Kraftträger (1) zwei benachbarte Löcher (2, 3, 4) hat, die auf verschiedenen Seiten der Bruch- oder Instabilitätszone des Knochens anzuordnen sind, und an denen der Kraftträger (1) ein Verstärkung (5, 6) hat, im Vergleich zu seiner Ausgestaltung an Löchern (2, 3, 4), die weiter entfernt von der Bruch- oder Instabilitätszone des Knochens anzuordnen sind, **dadurch gekennzeichnet, daß** die Knochenschrauben (36 bis 41) in den Löchern (2, 3, 4) fixierbar sind und die Verstärkung (5, 6) eine Verbreiterung des Kraftträgers (1 ) und/oder eine Verringerung der Querausdehnung des Loches (2, 3, 4) des Kraftträgers (1) und/oder einen Bereich mit festerem Werkstoff des Kraftträgers (1) aufweist.

2. Fixationssystem nach Anspruch 1, bei dem die Querschnittsvergrößerung eine Verdickung des Kraftträgers (1) aufweist.

3. Fixationssystem nach Anspruch 1 oder 2, bei dem der Kraftträger (1) an einem Loch (2, 3, 4), das weiter von der Bruch- oder Instabilitätszone des Knochens entfernt anzuordnen ist als ein anderes Loch (2, 3, 4), ebenfalls eine Verstärkung (5, 6) aufweist, die jedoch geringer als an einem Loch (2, 3, 4) ist, das näher an der Bruch- oder Instabilitätszone anzuordnen ist.

4. Fixationssystem nach Anspruch 3, bei dem der Kraftträger (1) an dem Loch (2, 3, 4), das am nächsten an der Bruch- oder Instabilitätszone des Knochens anzuordnen ist, eine Verstärkung (5, 6) aufweist, bei dem der Kraftträger (1) ferner an einem dem vorgenannten Loch (2, 3, 4) benachbarten Loch, das weiter von der Bruch- oder Instabilitätszone des Knochens anzuordnen ist, eine Verstärkung (5, 6) aufweist, die nur etwa halb so stark wie die ersterwähnte Verstärkung ist, und daß der Kraftträger (1) an einem noch weiter von der Bruch- oder Instabilitätszone entfernt anzuordnenden Loch (2, 3, 4) keine Verstärkung (5, 6) aufweist.

5. Fixationssystem nach einem der Ansprüche 1 bis 4, **dadurch gekennzeichnet, daß** mindestens zwei Löcher (2, 3, 4) schräg zueinander geneigt sind.

6. Fixationssystem nach Anspruch 5, bei dem die Achsen der beiden Löcher (2, 3, 4) auf der dem Knochen zuzuwendenden Seite des Kraftträgers (1) divergieren.

7. Fixationssystem nach Anspruch 6, bei dem die mindestens zwei schräg zueinander geneigten Löcher (2, 3, 4) auf verschiedenen Seiten eines Abschnittes des Kraftträgers (1) angeordnet sind, der einer Bruch- oder Instabilitätszone eines Knochens zuzuordnen ist.

8. Fixationssystem nach einem der Ansprüche 1 bis 7, bei dem mindestens ein Loch (2, 3, 4) schräg zum Kraftträger (1) geneigt ist.

9. Fixationssystem nach einem der Ansprüche 1 bis 8, bei dem mindestens zwei Löcher (2, 3, 4) des an einen Knochen modellierten Kraftträgers (1) schräg zueinander geneigt sind.

10. Fixationssystem nach einem der Ansprüche 1 bis 9, bei dem die Knochenschrauben (36 bis 41) unter verschiedenen Winkeln in die Löcher (2, 3, 4) des Kraftträgers (1) einsetzbar und fixierbar sind.

11. Fixationssystem nach einem der Ansprüche 1 bis 10, bei dem der Kraftträger ( 1 ) eine Knochenplatte, ein Knochennagel oder ein Fixateur ist.

## Claims

1. Fixing system for bones with a force-bearing device (1) with holes (2, 3, 4) and bone screws (36 to 41) which can be inserted into the holes (2, 3, 4), in which the force-bearing device (1) has two adjacent holes (2, 3, 4) which are to be arranged on different sides of the fracture or the area where the bone is weak and at which the force-bearing device (1) has a reinforcement (5, 6), compared to its form at holes (2, 3, 4) which are to be arranged further away from the fracture or the area where the bone is weak, **characterised in that** the bone screws (36 to 41) can be fixed into the holes (2, 3, 4) and the reinforcement (5, 6) has a widening of the force-bearing device (1) and/or a reduction of the transverse extension of the hole (2, 3, 4) of the force-bearing device (1) and/or a region of the force-bearing device (1) with more solid material.

2. Fixing system according to claim 1, in which the cross-sectional enlargement has a thickening of the force-bearing device (1).

3. Fixing system according to claim 1 or 2, in which the force-bearing device (1) also has a reinforcement (5, 6) at a hole (2, 3, 4) which is to be arranged further away from the fracture or the area where the bone is weak than another hole (2, 3, 4) and which is, however, smaller than at a hole (2, 3, 4) which is to be arranged nearer the fracture or the area where the bone is weak.

4. Fixing system according to claim 3, in which the force-bearing device has a reinforcement (5, 6) at the hole (2, 3, 4) which is to be arranged closest to the fracture or the area where the bone is weak, in which the force-bearing device (1) moreover has a reinforcement (5, 6) at a hole adjacent to the aforementioned hole (2, 3, 4) which is to be arranged further from the fracture or the area where the bone is weak and which is only approximately half as large as the first-mentioned reinforcement and that the force-bearing device (1) has no reinforcement (5, 6) at a hole (2, 3, 4) to be arranged even further away from the fracture or the area where the bone is weak.

5. Fixing system according to any of claims 1 to 4, **characterised in that** at least two holes (2, 3, 4) are inclined obliquely to one another.

6. Fixing system according to claim 5, in which the axes of the two holes (2, 3, 4) diverge on the side of the force-bearing device (1) facing the bone.

7. Fixing system according to claim 6, in which the at least two holes (2, 3, 4) inclined obliquely toward one another are arranged on different sides of a portion of the force-bearing device (1) which is to be associated with a fracture or the area where the bone is weak.

8. Fixing system according to any of claims 1 to 7, in which at least one hole (2, 3, 4) is inclined obliquely toward the force-bearing device (1).

9. Fixing system according to any of claims 1 to 8, in which at least two holes (2, 3, 4) of the force-bearing device (1) shaped on a bone are inclined obliquely toward one another.

10. Fixing system according to any of claims 1 to 9, in which the bone screws (36 to 41) can be inserted and fixed at different angles into the holes (2, 3, 4) of the force-bearing device (1).

11. Fixing system according to any of claims 1 to 10, in which the force-bearing device (1) is a bone plate, a bone pin or a fixator.

## Revendications

1. Système de fixation des os avec un support de force (1) ayant des trous (2, 3, 4) et des vis pour ostéosynthèse (36 à 41) pouvant être mises en place dans les trous (2, 3, 4), dans lequel le support de force (1) a deux trous (2, 3, 4) voisins qui doivent être disposés sur différents côtés de la zone de fracture ou d'instabilité de l'os, et sur lesquels le support de force (1) a un renfort (5, 6), en comparaison avec sa configuration sur les trous (2, 3, 4), qui doivent être disposés plus loin de la zone de fracture ou d'instabilité de l'os, **caractérisé en ce que** les vis pour ostéosynthèse (36 à 41) peuvent être fixées dans les trous (2, 3, 4) et **en ce que** le renfort (5, 6) présente un élargissement du support de force (1) et/ou une réduction de l'extension transversale du trou (2, 3, 4) du support de force (1) et/ou une zone avec un matériau plus solide du support de force (1).

2. Système de fixation selon la revendication 1, dans lequel l'augmentation de la section présente un épaississement du support de force (1).

3. Système de fixation selon la revendication 1 ou 2, dans lequel le support de force (1) présente, au niveau d'un trou (2, 3, 4) qui doit être disposé plus loin de la zone de fracture ou d'instabilité de l'os qu'un autre trou (2, 3, 4), également un renfort (5, 6) qui est cependant plus petit qu'au niveau d'un trou (2, 3, 4) qui doit être disposé plus près de la zone de fracture ou d'instabilité.

4. Système de fixation selon la revendication 3, dans lequel le support de force (1) présente un renfort sur le trou (2, 3, 4) qui doit être disposé le plus près de la zone de fracture ou d'instabilité de l'os, dans lequel le support de force (1) présente en outre, sur un trou qui est voisin du trou (2, 3, 4) précité et qui doit être disposé plus loin de la zone de fracture ou d'instabilité de l'os, un renfort (5, 6) qui est deux fois moins épais que le renfort mentionné en premier, et en ce que le support de force (1), sur un trou (2, 3, 4) devant être disposé encore plus loin de la zone de fracture ou d'instabilité, ne présente aucun renfort (5, 6).

5. Système de fixation selon une des revendications 1 à 4, **caractérisé en ce que** au moins deux trous (2, 3, 4) sont inclinés obliquement l'un par rapport à l'autre.

6. Système de fixation selon la revendication 5, dans lequel les axes des deux trous (2, 3, 4) divergent sur le côté du support de force (1) qui doit être tourné vers l'os.

7. Système de fixation selon la revendication 6, dans lequel les trous (2, 3, 4), au moins au nombre de deux, inclinés obliquement l'un par rapport à l'autre sont disposés sur différents côtés d'un tronçon du support de force (1) qui doit être affecté à une zone de fracture ou d'instabilité d'un os.

8. Système de fixation selon une des revendications 1 à 7, dans lequel au moins un trou (2, 3, 4) est incliné obliquement par rapport au support de force (1).

9. Système de fixation selon une des revendications 1 à 8, dans lequel au moins deux trous (2, 3, 4) du support de force (1) modelé sur un os sont inclinés l'un par rapport à l'autre.

10. Système de fixation selon une des revendications 1 à 9, dans lequel les vis pour ostéosynthèse (36 à 41) peuvent être mises en place et fixées sous différents angles dans les trous (2, 3, 4) du support de force (1).

11. Système de fixation selon une des revendications 1 à 10, dans lequel le support de force (1) est une plaque d'ostéosynthèse, un clou à os ou un fixateur.
